# EUROPÄISCHE PATENTSCHRIFT

(11) **EP 3 987 184 B1**
(45) Veröffentlichungstag und Bekanntmachung des Hinweises auf die Patenterteilung: **08.04.2026**
(21) Anmeldenummer: 20733592.8
(22) Anmeldetag: 17.06.2020
(51) Int. Cl.: F04D 7/04, F04D 15/00, F04D 15/02, F04D 29/66

(54) **KREISELPUMPE UND VERFAHREN ZUR ZUSTANDSERKENNUNG EINER KREISELPUMPE**
CENTRIFUGAL PUMP AND METHOD FOR STATUS DETECTION OF A CENTRIFUGAL PUMP
POMPE CENTRIFUGE ET PROCÉDÉ POUR RECONNAÎTRE L'ÉTAT D'UNE POMPE CENTRIFUGE

(30) Priorität: 18.06.2019 DE 102019004263
(43) Veröffentlichungstag der Anmeldung: 27.04.2022
(73) Patentinhaber: KSB SE & Co. KGaA, 67227 Frankenthal (DE)
(72) Erfinder: SERYCZYNSKI, Jakub, 67227 Frankenthal (DE)
(86) Internationale Anmeldenummer: PCT/EP2020/066721
(87) Internationale Veröffentlichungsnummer: WO 2020/254380

(56) Entgegenhaltungen:
- EP-A2- 2 538 085
- WO-A2-2010/145762
- JP-A- 2010 101 192
- JP-A- 2017 137 811
- US-A1- 2018 033 129

## Beschreibung

Die vorliegende Erfindung betrifft eine Kreiselpumpe und ein Verfahren zur Zustandserkennung einer Kreiselpumpe.

Kreiselpumpen werden in einer Vielzahl von Anwendungen verwendet um eine Flüssigkeit zu fördern. Dabei befinden sich insbesondere bei Brauchwasser oder Abwasser oftmals Verunreinigungen in dem zu fördernden Fluid, die unter Umständen zu einer Verstopfung oder einer ungewünschten Wirkungsbeeinträchtigung der Pumpe führen können. Typischerweise wickeln sich dabei Fremdelemente um eine Achse oder die Schaufeln des Laufrads, so dass die Leistungsfähigkeit der Pumpenanordnung nachlässt. In Extremfällen kann das Aufwickeln von Fremdgut um die Drehachse des Laufrads herum zu einer Blockade oder dergleichen führen.

Aus dem Stand der Technik, bspw. aus der WO 2008/119931 A2, ist ein Verfahren zur Erkennung eines solchen ungewünschten Zustands bekannt, bei dem die Stromaufnahme der Pumpe überwacht wird und bei Abweichungen von einem Normalwert auf eine Verstopfung geschlossen wird.

Die EP 2 538 085 A2 beschreibt eine Pumpenanordnung mit einer Pumpvorrichtung, bei der eine Diagnoseeinheit zur Ermittlung des Betriebszustands der Pumpvorrichtung vorgesehen ist.

Die JP 2010101192 A offenbart eine Vorrichtung zur Vorhersage einer Erosionsmenge in einem Turbinenlaufrad aufgrund von Kavitation durch erfassen eines Bildes eines Abschnitts, in dem Kavitation auftritt.

Es ist das Ziel der vorliegenden Erfindung eine möglichst einfache und zuverlässige Zustandserkennung bei Kreiselpumpen vorzusehen, damit auch sich bereits anbahnende Verstopfungszustände frühzeitig erkannt und entsprechende Gegenmaßnahmen eingeleitet werden können.

Dies gelingt mit einer Pumpenanordnung, die sämtliche Merkmale des Anspruchs 1 aufweist oder mithilfe eines Verfahrens, das sämtliche Verfahrensschritte nach Anspruch 10 ausführt.

Weitere vorteilhafte Ausgestaltungen der Erfindung bzw. weitere vorteilhafte Spezifizierungen des Verfahrens finden sich in den jeweiligen abhängigen Ansprüchen.

Nach dem Anspruch 1 umfasst die Pumpenanordnung zum Fördern einer Flüssigkeit, insbesondere zum Fördern eines Abwassers oder eines Brauchwassers, ein Pumpengehäuse mit einem Saugstutzen zum ansaugen der zu förderden Flüssigkeit, und ein in dem Pumpengehäuse rotierbar angeordnetes Laufrad zum Fördern der Flüssigkeit. Mindestens ein Sensor, insbesondere ein 3D-Sensor, zum Erfassen der Oberfläche des Laufrads ist vorhanden, wobei dieser mindestens eine Sensor am und/oder im Pumpengehäuse angeordnet ist. Durch das Vorsehen eines Sensors, dessen Erfassungsbereich auf das Laufrad gerichtet ist, kann in einer kontinuierlichen oder in einer intermittierenden Art und Weise ein bestimmter Abschnitt des Laufrads erfasst werden. Mithilfe einer Auswerteeinheit, die die erfassten Aufnahmen des Sensors auswertet, kann dann auf das Vorliegen eines Fehlerzustands, wie bspw. eine drohende Verstopfung oder eine bereits fortgeschrittenen Zopfbildung geschlossen werden.

Durch die optionale 3D-Funktionalität kann der Sensor Informationen über anhaftende oder vor dem Laufrad befindliche Gegenstände erhalten, so dass eine verlässliche Aussage über das Vorhandensein von unerwünschten Gegenständen getroffen werden kann.

Da es gerade bei der Förderung von Brauchwasser oder gar Abwasser nicht unüblich ist, dass in der dabei geförderten Flüssigkeit kleinere oder auch größere Gegenstände fester Form mitschwimmen, wird in der Regel nicht bereits das einmalige Erfassen eines solchen Gegenstands vor dem Laufrad zu einem Fehlerfall führen. Vielmehr ist es dabei für eine Zopfbildung charakteristisch, dass ein Festkörper über mehrere Umdrehungen hinweg denselben oder einen ähnlichen Bereich einer Oberfläche des Laufrads verdeckt und nicht mit der gepumpten Flüssigkeit abgeführt worden ist.

Nach einer Fortbildung der Erfindung kann vorgesehen sein, dass der Sensor dazu ausgelegt ist, einen Abstand zum Laufrad zu bestimmen, vorzugsweise mithilfe der Timeof-Flight-Technologie (auch: ToF-Technologie).

Die ToF-Technologie basiert im Wesentlichen auf der Laufzeitmessung eines ausgesandten Signals, sodass man durch die gemessene Signallaufzeit des ausgesandten Signals ziemlich exakt den Abstand eines Objekts zu einem Signalursprung berechnen kann. Vorliegend ist dieses Verfahren von großem Vorteil, da es für die ToF-Technik besonders widerstandsfähige Sensoren gibt, die sich auch für den Einbau in einer Kreiselpumpe eignen und den dort herrschenden widrigen Umständen standhalten können.

Wie bereits vorstehend kurz erläutert, ist die Abstandsbestimmung zwischen Sensor und Laufrad bzw. eines sich daran anhaftenden Objekts eine wirksame Möglichkeit, das Vorhandensein von um die Rotationsachse des Laufrads mitgezogenen Gegenständen zu erfassen.

Als vorteilhafte Alternative kann vorgesehen sein, dass der Sensor dazu ausgelegt ist, einen Abstand zum Laufrad mithilfe des Phasendifferenzverfahrens zu messen.

Als weitere Alternative bietet sich vorteilhafterweise die Frequency-modulated-continous-wave-Technologie (FMCW oder Frequenzmodulierte Dauerwelle) an.

Gemäß einer optionalen Modifikation der vorliegenden Erfindung kann vorgesehen sein, dass der Sensor ein optischer Sensor, ein optischer 2D-Sensor, ein optischer 3D-Sensor, ein 2D-Ultraschallsensor, ein 3D-Ultraschallsensor, ein MIMO-Radarsensor (Multiple-Input Multiple-Output Radarsensor), ein 2D-Laser-Abstandssensor und/oder ein 3D-Laser-Abstandssensor vorzugsweise nach dem Triangulationsprinzip ist.

Nach einer bevorzugten Ausführung der Erfindung kann das Laufrad an seiner Oberfläche mit einem charakteristischen Muster versehen sein, das bei einer bestimmten Drehposition des Laufrads durch den Sensor erfassbar ist, vorzugsweise ist dabei das Muster ein 2D-Muster oder ein 3D-Muster.

Durch das Anbringen eines Musters auf dem Laufrad, kann es dem Sensor erleichtert werden, einen bestimmten Bereich des Laufrads optisch zu erfassen bzw. erleichtert sich dadurch die Auswertung der vom Sensor erzeugten Aufnahmen. So kann ein auf den verwendeten Typen des Sensors abgestimmtes charakteristisches Muster sehr viel leichter erkannt werden, auch wenn die gemachten Aufnahmen nicht unter optimalen Bedingungen angefertigt worden sind oder durch in der zu fördernden Flüssigkeit vorhandene Teile teilweise verdeckt ist. So wird das zu fördernde Fluid typischerweise mit absorbierenden Partikeln versetzt sein, die die Aufnahmequalität verschlechtern. Zudem kann trotz einer eventuell erfolgenden Beleuchtung des untersuchten Bereichs des Laufrads ein trübes oder verdrecktes zu förderndes Fluid die Aufnahmequalität signifikant verringern. Dabei ist es dann von Vorteil, wenn das charakteristische Muster verwendet wird, da dies auch bei nicht-optimalen Bedingungen einen deutlich besseren Rückschluss auf das Vorhandensein von Verstopfungen oder dergleichen erleichtert.

Demnach kann also vorgesehen sein, dass ferner ein Leuchtmittel in und/oder am Pumpengehäuse vorhanden ist, um einen Erfassungsbereich des Sensors auszuleuchten. Insbesondere ist dies bei einem optischen Sensor hilfreich, der zum Erfassen eines optischen Bilds eine ausreichende Beleuchtung erfordert.

Nach einer optionalen Modifikation der Erfindung kann dabei vorgesehen sein, dass das Leuchtmittel dazu ausgelegt ist, intermittierende Lichtimpulse auszugeben, wobei die intermittierend abgegebenen Lichtimpulse mit einer Pumpendrehzahl synchronisiert sind.

So kann der Lichtimpuls nur dann erzeugt werden, wenn sich der interessierende Bereich des Laufrads im Erfassungsbereich des Sensors befindet, und nach einem Verlassen dieses Bereichs wieder deaktiviert werden. Die Beleuchtung des Aufnahmebereichs ist vorzugsweise mit der Pumpendrehzahl gekoppelt, so dass keine komplizierte Steuerung erforderlich ist.

Erfindungsgemäß ist vorgesehen, dass eine Mikropumpe zum Spülen einer Sensoroberfläche des Sensors vorhanden ist, um vor der Sensorfläche sich ansammelnde Rückstände aktiv zu entfernen. Diese Mikropumpe ist ein im Inneren des 10 Pumpengehäuses angeordnetes Bauteil, das z. B. eine Teilmenge der zu fördernden Flüssigkeit der Pumpe gezielt auf die Sensoroberfläche des Sensors führt, so dass sich dort keine unerwünschten Ablagerungen festsetzen können. Alternativ erfolgt die Beaufschlagung der Sensoroberfläche nicht kontinuierlich sondern nur bei Bedarf. Die Mikropumpe ist also im Endeffekt dazu ausgelegt, die Sensoroberfläche freizuspritzen, so 15 dass sich dort keine störenden Rückstände ablagern können oder dort bereits vorhandene Rückstände entfernt werden können. Die Mikropumpe kann aber auch in dem Sensor integriert ausgebildet sein.

Eine weitere alternative oder zusätzliche Möglichkeit zum Freihalten der Sensoroberfläche ist erfindungsgemäß mit Hilfe eines Ultraschall-Mikroaktors gegeben, der dazu ausgelegt ist, um ein Außengehäuse bzw. eine Sensorfläche des Sensors in eine Ultraschall-Rüttelbewegung zu versetzen. Dies führt dazu, dass eine sich daran anlagernde Schmutzschicht aufgebrochen oder sich eine Schmutzschicht gar nicht erst ausbilden kann. Auch so wird das Anlagern von unerwünschten Rückständen auf der Oberfläche des Sensors verhindert.

Erfindungsgemäß kann ferner vorgesehen sein, dass die Pumpe einen weiteren Zuführstutzen auf der Saugseite der Pumpe zum Zuführen von Reinwasser aufweist, um eine eventuelle Trübheit des zu fördernden Fluids im Bereich des Laufrads zumindest kurzzeitig zu verringern. Oftmals behindert die Trübheit der durch die Pumpe strömenden Flüssigkeit die Leistungsfähigkeit des Sensors, da die damit gemachten Aufnahmen nicht oder nur eingeschränkt brauchbar sein können. Damit eine Zustandserkennung aber auch bei einer sehr verdreckten Flüssigkeit einwandfrei funktioniert, ist vorgesehen, dass neben dem Saugstutzen zum Ansaugen der zu pumpenden Flüssigkeit ein auf der Saugseite der Pumpe angeordneter Zuführstutzen vorhanden ist, über den bei Bedarf, bspw. während oder kurz vor einer Aufnahme durch den Sensor, eine klare Flüssigkeit, bspw. klares Wasser, in die Pumpe eingeführt wird. Dies führt dazu, dass die Trübheit der Flüssigkeit zumindest während der von dem Sensor getätigten Aufnahme vorübergehend verringert wird, so dass die in dieser Zeit gemachten Aufnahmen aussagekräftiger sind.

Nach einer Fortbildung der Erfindung kann vorgesehen sein, dass der Zuführstutzen dazu ausgelegt ist, eine klare Flüssigkeit in Abhängigkeit der Pumpendrehzahl zuzuführen. Dies ermöglicht, dass, wenn eine Aufnahme des Sensors erfolgt, die klare Flüssigkeit zu einer Verringerung der Trübheit um den Aufnahmebereich führt und so bessere Aufnahmen getätigt werden können.

Dabei ist von Vorteil, wenn der Zuführstutzen auch im Pumpengehäuse angebracht ist, da dann der Abstand vom Aufnahmebereich gering und eine Durchmischung einer zugeführten klaren Flüssigkeit noch nicht in dem Maß stattgefunden hat, wie es bei einer entfernteren Anordnung des Zuführstutzens der Fall gewesen wäre.

Nach einer Fortbildung der Erfindung kann vorgesehen sein, dass die Pumpenanordnung eine Steuereinheit aufweist, die mit dem Sensor verbunden ist und dazu ausgelegt ist, von dem Sensor erhaltene Bilddaten auszuwerten und ausgehend hiervon auf einen Fehlerfall, insbesondere eine Verstopfung, eine Zopfbildung, eine Kavitation, mechanische Schäden und/oder Vibrationen zu schließen.

Dies geschieht bspw. dann, wenn in der Aufnahme detektiert wird, dass nicht etwa das Laufrad zu sehen ist sondern ein zwischen Laufrad und Sensor angeordnetes Objekt.

Vorzugsweise ist vorgesehen, dass die Auswertung einer durch den Sensor gemachten Aufnahme mit Hilfe von künstlicher Intelligenz durchgeführt wird, vorzugsweise auf Grundlage von deep-learning. Dies unterstützt die Zuverlässigkeit bei der Erkennung eines drohenden oder gar bereits herrschenden Fehlerzustands, wie Zopfbildung oder Verstopfung.

Die Erfindung betrifft zudem ein Verfahren zum Erkennen eines Fehlerfalls einer Pumpenanordnung, insbesondere einer Kreiselpumpenanordnung, nach einer der vorstehend beschriebenen Varianten, wobei in dem Verfahren der Sensor Aufnahmen des Laufrads erzeugt, und die vom Sensor erzeugten Daten bzw. Aufnahmen des Laufrads durch eine Steuereinheit ausgewertet werden, um auf den Fehlerfall, insbesondere eine Verstopfung, eine Zopfbildung, eine Kavitation, mechanische Schäden und/oder Vibrationen zu schließen.

Vorteilhaft an diesem neuen Ansatz ist, dass die Komponenten zur automatisierten Bildverarbeitung preisgünstig und sehr leistungsstark sind und es nicht mehr erforderlich ist - wie im Stand der Technik üblich - eine eigene Logik aufzubauen, die die Stromaufnahme der Pumpe auf Abweichungen überwacht.

Eine der beanspruchten Optionen sieht vor, dass kurz vor dem Erstellen der Aufnahmen des Laufrads Reinwasser auf der Saugseite der Pumpe hinzugegeben wird, um eine eventuelle Trübheit der in der Umgebung des Laufrads befindlichen Flüssigkeit für den Zeitpunkt der Aufnahmen zu verringern. Vorzugsweise kann vorgesehen sein, dass die Aufnahmen des Sensors einen bestimmten Bereich des Laufrads wiedergeben, der mit einem charakteristischen Muster versehen ist, so dass die Beurteilung über das Vorliegen eines Verstopfungsfalls oder einer Zopfbildung leichter durchführbar ist, da das Muster in einem solchen Fall gar nicht oder nur erschwert erkennbar ist. Dazu kann ein spezielles 2D oder 3D- Muster auf das Laufrad aufgebracht werden, so dass die Aufnahmen einfacher auswertbar sind.

Darüber hinaus kann in dem Verfahren vorgesehen sein, dass das Erstellen der Aufnahmen mit der Drehzahl des Laufrads synchronisiert ist, um vorzugsweise in einer intermittierenden Art und Weise einen bestimmten Abschnitt oder mehrere bestimmte Abschnitte des Laufrads mit dem Sensor durchgehend zu erfassen.

Weiter kann vor einem Aufnehmen auch der aufzunehmende Bereich des Laufrads mit einer Beleuchtungsquelle beleuchtet werden, so dass die Aufnahmen eine bessere und leichtere Auswertung, insbesondere bei der Umsetzung des Sensors als optischer Sensor, zulassen.

Weiter kann nach der Erfindung vorgesehen sein, dass Aktivitäten eines Bedienpersonals der Pumpe wie das Ein- oder Ausschalten der Pumpe und/oder eine Fehlerquittierung mithilfe des Sensors überwacht werden, um einen verlässlicheren Ist- Zustand der Pumpe zu erhalten.

Dies ist insbesondere für die Verringerung einer Gesamtfehlerwahrscheinlichkeit von Bedeutung, bei der oftmals kaputte Sensoren den Ist-Zustand nicht korrekt wiedergeben. Durch das erfindungsgemäße Verfahren wird die Wahrscheinlichkeit hierfür weiter verringert.

Weitere Vorteile, Einzelheiten und Merkmale werden aus der nachfolgenden Beschreibung der Figuren ersichtlich. Es zeigen die
- Fig. 1: eine Schnittansicht einer erfindungsgemäßen Pumpenanordnung mit dem Sensor, die
- Fig. 2: eine Schnittansicht einer weiteren erfindungsgemäßen Pumpe mit dem Sensor und die
- Fig. 3: eine Schnittansicht einer weiteren erfindungsgemäßen Pumpe mit dem Sensor

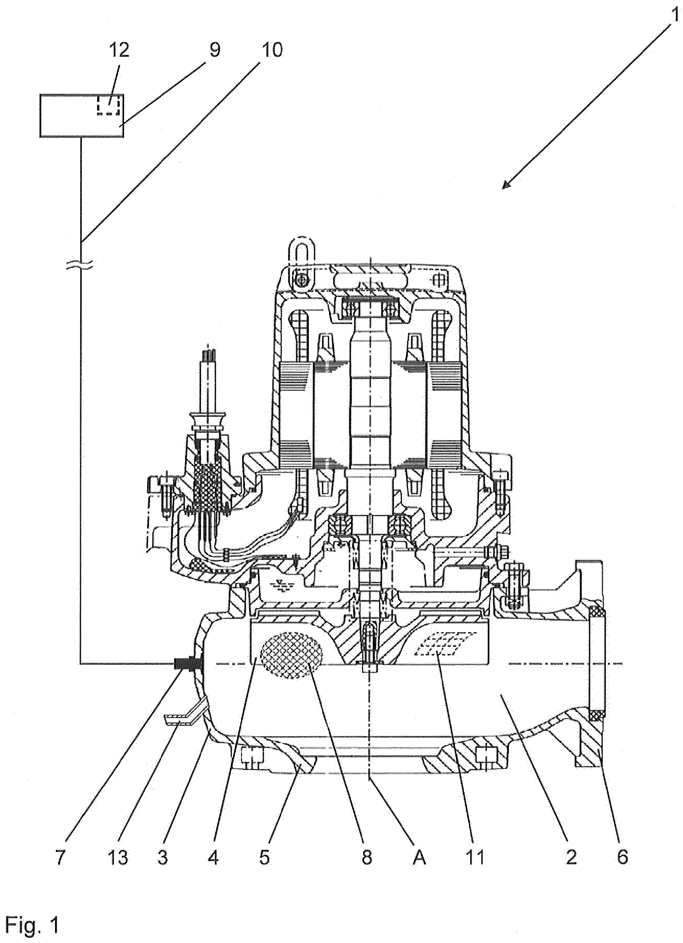

Die Fig. 1 zeigt eine vertikal aufgestellte Pumpenanordnung 1 mit einem eine Strömungskammer 2 aufweisenden Pumpengehäuse 3 und einem darin angeordneten Laufrad 4, das bei Rotation um seine Drehachse A eine Flüssigkeit von seinem Saugstutzen 5 zu seinem Druckstutzen 6 fördert.

Darüber hinaus ist in und/oder an dem Pumpengehäuse 3 ein Sensor 7 vorgesehen, der auf eine Oberfläche des Laufrads 4 gerichtet ist. So kann der Sensor 7 erkennen, ob ein vor der Laufradoberfläche angeordnetes Objekt 8 vorhanden ist, das die Leistungsfähigkeit der Pumpe 1 verringert. Typischerweise kann damit eine sich drohende Verstopfung der Pumpe 1 erkannt oder auf eine Zopfbildung geschlossen werden.

Eine Zopfbildung bedeutet dabei das Anhaften von einem Festkörper 8 an dem Laufrad 4, der auch bei einer fortwährenden Rotation nicht auf die Druckseite abgegeben wird. Durch das permanente Mitziehen entlang der zu fördernden Flüssigkeit haften sich hieran andere freischwimmende Elemente an, so dass der Zopf wächst und die Leistungsfähigkeit der Pumpe 1 immer stärker beeinträchtigt. Dies kann mitunter zu einem vollständigen Verstopfen der Pumpe 1 führen.

Um einen solchen Zustand möglichst frühzeitig zu erkennen, werden die Aufnahmen bzw. die vom Sensor 7 erfassten Informationen an eine Steuereinheit 9 über ein Kabel 10 oder auch kabellos gesendet, so dass dort eine entsprechende Auswertung stattfinden kann. Dies geschieht vorteilhafterweise mit Hilfe von künstlicher Intelligenz, wobei hier auch auf deep-learning-Technologie zurückgegriffen werden kann.

Dabei wird also die zu erwartende Aufnahme der Laufradoberfläche mit der tatsächlichen Aufnahme abgeglichen, wobei hieraus abgeleitet werden kann, ob eine Zopfbildung entsteht oder gar eine Verstopfung vorliegt.

Um einen besseren Abgleich zu erhalten, ist in der dargestellten Ausführungsform ein Muster 11 auf die Oberfläche des Laufrads 4 angebracht worden, das für den Sensor 7 besonders gut erfassbar ist. Nimmt der Sensor 7 nun das Muster 11 auf, kann besonders gut ein davor befindliches Objekt 8 erkannt werden, was die Zuverlässigkeit einer Fehlerzustandserkennung verbessert.

Das Bezugszeichen 12 zeigt dabei symbolhaft eine Kommunikationsschnittstelle, die bei einer optionalen Modifikation der Erfindung dazu genutzt werden kann, die Ergebnisse der Auswertungen weiter zu übertragen.

Damit eine Zustandserkennung auch bei einer sehr verdreckten Flüssigkeit einwandfrei funktioniert, ist als eine beanspruchte Option vorgesehen, dass neben dem Saugstutzen 5 zum Ansaugen der zu pumpenden Flüssigkeit ein auf der Saugseite der Pumpenanordnung 1 angeordneter Zuführstutzen 13 vorhanden ist, über den bei Bedarf, bspw. während oder kurz vor einer Aufnahme durch den Sensor 7, eine klare Flüssigkeit, bspw. klares Wasser, in die Pumpe 1 eingeführt wird.

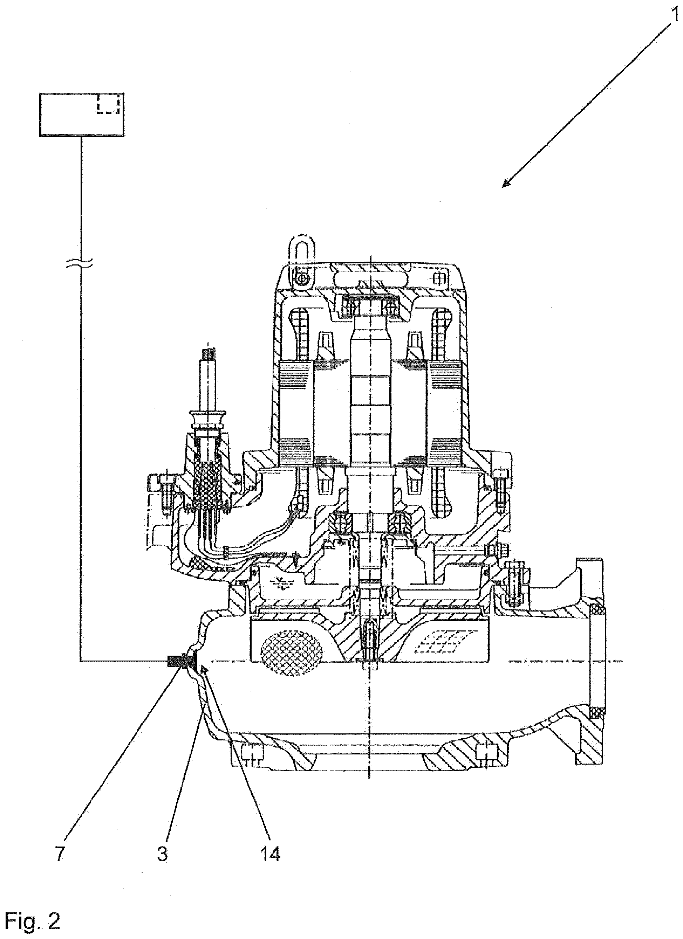

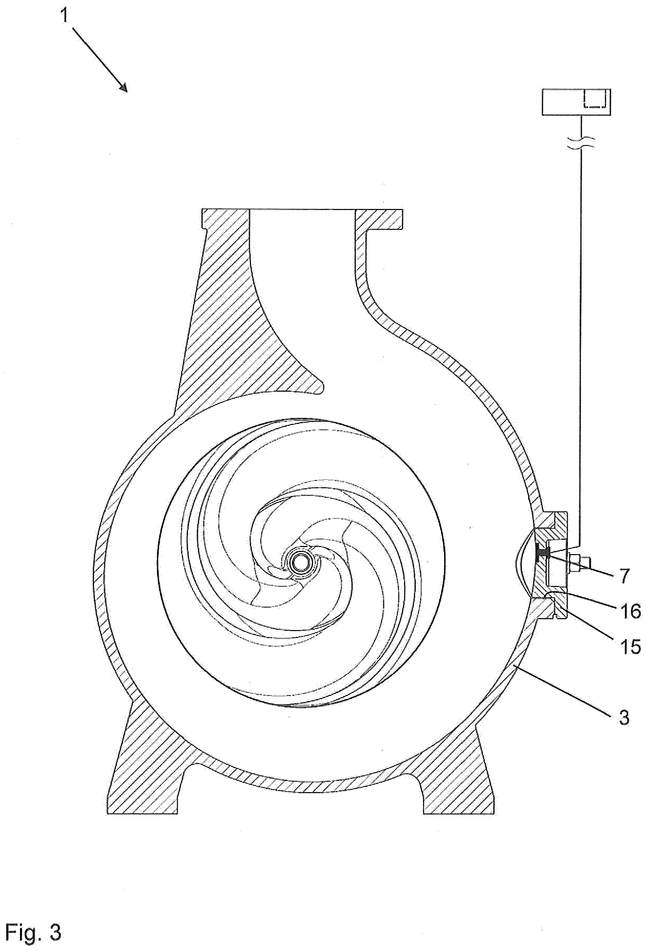

Die Fig. 2 zeigt eine weitere Ausführungsform der Pumpenanordnung 1, bei der der Sensor 7 in einem aus der Strömungskammer 2 zurückgesetzten Bereich 14 angeordnet ist. Somit ist der Sensor 7 mit dem Fördermedium in Berührung, jedoch besser vor abrasivem Verschleiß, mechanischen Schlägen durch die im Medium enthaltenen Feststoffe sowie Verschmutzung und Sedimentierung geschützt. Der zurückgesetzte Bereich wird in der Fig. 2 als Wölbung im Pumpengehäuse gezeigt. Alternativ kann der zurückgesetzte Bereich 14 durch mechanische Bearbeitung, insbesondere durch eine spanabhebende Bearbeitung, der Innenseite des Pumpengehäuses 3 erzeugt werden. Die Fig. 3 zeigt eine vertikal aufgestellte Pumpenanordnung 1, bei welcher der Sensor 7 in einem Deckel 15 zum Verschließen eines sogenannten Putzloches 16 angeordnet ist. Dadurch kann auf eine Bohrung im Pumpengehäuse 3 verzichtet werden.

Es versteht sich, dass in dem Deckel 15 ein zurückgesetzter Bereich, ähnlich dem in der Fig. 2 gezeigten Bereich 14 vorgesehen sein kann. Ferner ist es möglich, dass die in den Fig. 1 und 2 gezeigten Pumpenanordnungen 1 mit einem Deckel 15 gemäß der Fig. 3 ausgestattet sein kann.

Um den Erfassungsbereich des Sensors 7 auszuleuchten, kann ein in den Figuren nicht dargestelltes Leuchtmittel in und/oder am Pumpengehäuse 3 oder an dem Sensor 7 selbst vorgesehen sein.

Eine Platzierung des Sensors 7, des Leuchtmittels und/oder der Mikropumpe kann zudem auch an anderen dafür geeigneten Stellen im bzw. am Pumpengehäuse 3 vorgesehen werden, beispielsweise nahe dem Saugstutzen 5 und/oder nahe dem Druckstutzen 6. Die Figuren 1 bis 3 zeigen jeweils eine einstufige Pumpenanordnung mit einem in radialer Richtung ausstoßendem Laufrad 4. Die Erfindung kann beispielsweise auch in einer Pumpenanordnung 1 vorgesehen werden, die mehrstufig ausgebildet ist, und/oder mit einem in axialer oder halbaxialer Richtung ausstoßendem Laufrad 4 versehen ist.

Der zu der Fig. 1 beschriebene Zuführstutzen 13 kann auch in den anderen Ausführungsformen von Pumpenanordnungen 1 verwendet werden.

## Patentansprüche

1. Pumpenanordnung (1) zum Fördern einer Flüssigkeit, insbesondere zum Fördern eines Abwassers oder eines Brauchwassers, umfassend:
ein Pumpengehäuse (3) mit einem Saugstutzen (5) zum Ansaugen der zu förderden Flüssigkeit,
ein in dem Pumpengehäuse (3) um eine Drehachse (A) rotierbar angeordnetes Laufrad (4) zum Fördern der Flüssigkeit, und
mindestens einen Sensor (7), insbesondere einen 2D- oder einen 3D-Sensor, zum Erfassen der Oberfläche des Laufrads (4), wobei der mindestens eine Sensor (7) am und/oder im Pumpengehäuse (3) angeordnet ist,
**dadurch gekennzeichnet,**
**dass** die Pumpenanordnung (1) eine Mikropumpe zum Spülen einer Sensoroberfläche des Sensors (7) umfasst, um vor der Sensorfläche sich ansammelnde Rückstände aktiv zu entfernen, und/oder
**dass** die Pumpenanordnung (1) einen Ultraschall-Mikroaktor umfasst, um ein Außengehäuse bzw. eine Sensorfläche des 3D-Sensors (7) in eine Ultraschall-Rüttelbewegung zu versetzen, damit eine sich daran anlagernde Schmutzschicht aufgebrochen oder sich eine Schmutzschicht gar nicht erst ausbilden kann, und/oder
**dass** die Pumpenanordnung (1) einen weiteren Zuführstutzen (13) auf der Saugseite der Pumpe (1) umfasst, zum Zuführen von Reinwasser, um eine eventuelle Trübheit des zu fördernden Fluids im Bereich des Laufrads (4) zumindest kurzzeitig zu verringern.

2. Pumpenanordnung (1) nach Anspruch 1, wobei der Sensor (7) dazu ausgelegt ist, einen Abstand zum Laufrad (4) zu bestimmen, vorzugsweise mithilfe der Time-of-Flight-Technologie.

3. Pumpenanordnung (1) nach Anspruch 1, wobei der Sensor (7) dazu ausgelegt ist, einen Abstand zum Laufrad (4) mithilfe des Phasendifferenzverfahrens zu bestimmen.

4. Pumpenanordnung (1) nach Anspruch 1, wobei der Sensor (7) dazu ausgelegt ist, einen Abstand zum Laufrad (4) mithilfe der Frequency-modulated-continous-wave-Technologie zu bestimmen.

5. Pumpenanordnung (1) nach einem der vorhergehenden Ansprüche, wobei der Sensor (7) ein optischer Sensor, ein optischer 2D-Sensor, ein optischer 3D-Sensor, ein 2D-Ultraschallsensor, ein 3D-Ultraschallsensor, ein MIMO-Radarsensor (Multiple-Input Multiple-Output Radarsensor), ein 2D-Laser-Abstandssensor und/oder ein 3D-Laser-Abstandssensor vorzugsweise nach dem Triangulationsprinzip ist.

6. Pumpenanordnung (1) nach einem der vorhergehenden Ansprüche, wobei das Laufrad (4) an seiner Oberfläche mit einem charakteristischen Muster (11) versehen ist, das bei einer bestimmten Drehposition des Laufrads (4) durch den Sensor (7) erfassbar ist, vorzugsweise ist dabei das Muster (11) ein 2D-Muster oder ein 3D-Muster.

7. Pumpenanordnung (1) nach einem der vorhergehenden Ansprüche, wobei ferner ein Leuchtmittel in und/oder am Pumpengehäuse (3) oder an dem Sensor (7) selbst vorgesehen ist, um einen Erfassungsbereich des Sensors (7) auszuleuchten.

8. Pumpenanordnung (1) nach Anspruch 7, wobei das Leuchtmittel dazu ausgelegt ist, intermittierende Lichtimpulse auszugeben, wobei die intermittierend abgegebenen Lichtimpulse mit einer Pumpendrehzahl synchronisiert sind.

9. Pumpenanordnung (1) nach einem der vorhergehenden Ansprüche, ferner umfassend eine Steuereinheit (9), die mit dem Sensor (7) verbunden ist und dazu ausgelegt ist, von dem Sensor (7) erhaltene Daten auszuwerten und ausgehend hiervon auf einen Fehlerfall, insbesondere eine Verstopfung, eine Zopfbildung, eine Kavitation, mechanischen Schäden und/oder Vibrationen zu schließen.

10. Verfahren zum Erkennen eines Fehlerfalls in einer Pumpenanordnung (1) nach einem der vorhergehenden Ansprüche, wobei in dem Verfahren:
der Sensor (7), insbesondere ein 2D- oder ein 3D-Sensor, Aufnahmen des Laufrads (4) erzeugt, und die vom Sensor (7) erzeugten Daten des Laufrads (4) durch eine Steuereinheit (9) ausgewertet werden, um auf den Fehlerfall, insbesondere eine Verstopfung, eine Zopfbildung, eine Kavitation, mechanische Schäden und/oder Vibrationen zu schließen,
**dadurch gekennzeichnet,**
**dass** die Sensoroberfläche des Sensors (7) gespült wird, um sich vor der Sensorfläche ansammelnde Rückstände aktiv zu entfernen, und/oder
**dass** ein Außengehäuse bzw. eine Sensorfläche des 3D-Sensors (7) in eine Ultraschall-Rüttelbewegung versetzt wird, damit eine sich daran anlagernde Schmutzschicht aufgebrochen oder sich eine Schmutzschicht gar nicht erst ausbilden kann, und/oder
**dass** kurz vor dem Erstellen der Aufnahmen des Laufrads (4) Reinwasser auf der Saugseite der Pumpenanordnung (1) hinzugegeben wird, um eine eventuelle Trübheit der in der Umgebung des Laufrads (4) befindlichen Flüssigkeit für den Zeitpunkt der Aufnahmen zu verringern.

11. Verfahren nach Anspruch 10, wobei die Aufnahmen des Sensors (7) einen bestimmten Bereich des Laufrads (4) wiedergeben, der mit einem charakteristischen Muster (11) versehen ist, so dass die Beurteilung über das Vorliegen eines Verstopfungsfalls oder einer Zopfbildung leichter durchführbar ist, da das Muster (11) in einem solchen Fall gar nicht oder nur erschwert erkennbar ist.

12. Verfahren nach einem der Ansprüche 10 oder 11, wobei das Erstellen der Aufnahmen mit der Drehzahl des Laufrads (4) synchronisiert ist, um vorzugsweise in einer intermittierenden Art und Weise einen bestimmten Abschnitt oder mehrere bestimmte Abschnitte des Laufrads (4) mit dem Sensor (7) durchgehend zu erfassen.

13. Verfahren nach einem der vorhergehenden Ansprüche 10 bis 12, wobei Aktivitäten eines Bedienpersonals der Pumpenanordnung (1) wie das Ein- oder Ausschalten der Pumpenanordnung (1) und/oder eine Fehlerquittierung mithilfe des Sensors (7) überwacht werden, um einen verlässlicheren Ist- Zustand zu erhalten.

## Claims

1. Pump arrangement (1) for conveying a liquid, in particular for conveying waste water or process water, comprising:
a pump housing (3) comprising an intake connection (5) for drawing in the liquid being conveyed,
an impeller (4) rotatably arranged about a centre of rotation (A) in the pump housing (3) for conveying the liquid, and
at least one sensor (7), in particular a 2D or a 3D sensor, for capturing the surface of the impeller (4), the at least one sensor (7) being arranged on and/or in the pump housing (3),
**characterized**
**in that** the pump arrangement (1) comprises a micro-pump for rinsing a sensor surface of the sensor (7), so that residues accumulating in front of the sensor surface are actively removed, and/or
**in that** the pump arrangement (1) comprises an ultrasound micro-actuator in order to start an ultrasound shaking motion in an outside housing or a sensor surface of the 3D sensor (7), so that a layer of dirt which is deposited thereupon is broken up or a layer of dirt is prevented from forming in the first place, and/or
**in that** the pump arrangement (1) comprises a further supply connection (13) on the suction side of the pump (1) for supplying clean water, so that any possible murkiness in the fluid being conveyed is reduced, at least briefly, in the region of the impeller (4).

2. Pump arrangement (1) according to Claim 1, wherein the sensor (7) is designed to determine a distance from the impeller (4), preferably with the help of the time-of-flight technology.

3. Pump arrangement (1) according to Claim 1, wherein the sensor (7) is designed to determine a distance from the impeller (4) with the help of the phase-difference method.

4. Pump arrangement (1) according to Claim 1, wherein the sensor (7) is designed to determine a distance from the impeller (4) with the help of frequency-modulated continuous wave technology.

5. Pump arrangement (1) according to one of the preceding claims, wherein the sensor (7) is an optical sensor, an optical 2D sensor, an optical 3D sensor, a 2D ultrasound sensor, a 3D ultrasound sensor, a MIMO (multiple-input multiple-output) radar sensor, a 2D laser distance sensor and/or a 3D laser distance sensor, preferably based on the triangulation principle.

6. Pump arrangement (1) according to one of the preceding claims, wherein the impeller (4) is provided with a characteristic pattern (11) on its surface, which can be detected by the sensor (7) in a particular rotational position of the impeller (4); the pattern (11) in this case is preferably a 2D pattern or a 3D pattern.

7. Pump arrangement (1) according to one of the preceding claims, wherein a lighting means is provided in addition in and/or on the pump housing (3) or on the sensor (7) itself, in order to illuminate a detection range of the sensor (7).

8. Pump arrangement (1) according to Claim 7, wherein the lighting means is designed to emit intermittent light impulses, wherein the intermittently emitted light impulses are synchronized with a pump speed.

9. Pump arrangement (1) according to one of the preceding claims, in addition comprising a control unit (9) which is connected to the sensor (7) and is designed to analyse data received from the sensor (7) and to infer from this that there is a fault, in particular a blockage, plaiting, cavitation, mechanical damage and/or vibrations.

10. Method for detecting a fault in a pump arrangement (1) according to one of the preceding claims, wherein in the method:
the sensor (7), in particular a 2D or a 3D sensor produces images of the impeller (4), and the data on the impeller (4) produced by the sensor (7) are analysed by a control unit (9), in order to infer a fault, in particular a blockage, plaiting, cavitation, mechanical damage and/or vibrations,
**characterized**
**in that** the sensor surface of the sensor (7) is rinsed, so that residues accumulating in front of the sensor surface are actively removed, and/or
**in that** an ultrasound shaking motion is started in an outside housing or a sensor surface of the 3D sensor (7), so that a layer of dirt which is deposited thereupon is broken up or a layer of dirt is prevented from forming in the first place, and/or
**in that** just before the images of the impeller (4) are captured, clean water is added on the suction side of the pump arrangement (1), in order to reduce any murkiness in the liquid present in the vicinity of the impeller (4) for the time the images are taken.

11. Method according to Claim 10, wherein the images taken by the sensor (7) reproduce a particular region of the impeller (4) which is provided with a characteristic pattern (11), so that the assessment of the presence of a blockage or plaiting is easier to carry out, since the pattern (11) cannot be identified in such a case, or only with some difficulty.

12. Method according to either of Claims 10 and 11, wherein the capture of the images is synchronized with the speed of the impeller (4), so that a particular portion, or multiple particular portions, of the impeller (4) can be continuously captured using the sensor (7) preferably in an intermittent manner.

13. Method according to one of the preceding Claims 10 to 12, wherein activities of a staff member operating the pump arrangement (1), such as the switching-on or off of the pump arrangement (1) and/or an error acknowledgement, are monitored with the help of the sensor (7), in order to obtain a more reliable current state.

## Revendications

1. Ensemble pompe (1) pour refouler un liquide, en particulier pour refouler des eaux usées ou des eaux sanitaires, comprenant :
un carter de pompe (3) doté d'un raccord d'aspiration (5) pour aspirer le liquide à refouler,
une roue à aubes (4) disposée dans le carter de pompe (3) de manière à pouvoir tourner autour d'un axe de rotation (a), pour refouler le liquide, et
au moins un capteur (7), en particulier un capteur 2D ou 3D, destiné à détecter la surface de la roue à aubes (4), ledit au moins un capteur (7) étant disposé sur et/ou dans le carter de pompe (3),
**caractérisé**
**en ce que** l'ensemble pompe (1) comprend une micropompe pour rincer une surface de détection du capteur (7) afin d'éliminer activement des résidus s'accumulant devant la surface de détection, et/ou
**en ce que** l'ensemble pompe (1) comprend un micro-actionneur à ultrasons pour amener un boîtier extérieur ou une surface de capteur du capteur 3D (7) à effectuer un mouvement de secousse par ultrasons afin qu'une couche de salissure déposée sur celui-ci soit désagrégée ou que la formation d'une couche de salissure soit impossible, et/ou
**en ce que** l'ensemble pompe (1) présente, du côté aspiration de la pompe (1), un autre raccord d'alimentation (13) pour l'alimentation en eau propre, afin de réduire au moins brièvement une éventuelle turbidité du fluide à refouler dans la zone de la roue à aubes (4).

2. Ensemble pompe (1) selon la revendication 1, dans lequel le capteur (7) est configuré pour déterminer une distance par rapport à la roue à aubes (4), de préférence à l'aide de la technologie du temps de vol.

3. Ensemble pompe (1) selon la revendication 1, dans lequel le capteur (7) est conçu pour déterminer une distance par rapport à la roue à aubes (4) à l'aide du procédé de différence de phase.

4. Ensemble pompe (1) selon la revendication 1, dans lequel le capteur (7) est configuré pour déterminer une distance par rapport à la roue à aubes (4) à l'aide de la technologie à ondes entretenues modulées en fréquence.

5. Ensemble pompe (1) selon l'une des revendications précédentes, dans lequel le capteur (7) est un capteur optique, un capteur optique 2D, un capteur optique 3D, un capteur à ultrasons 2D, un capteur à ultrasons 3D, un capteur radar MIMO (capteur radar Multiple-Input Multiple-Output), un capteur de distance laser 2D et/ou un capteur de distance laser 3D, de préférence fondé sur le principe de la triangulation.

6. Ensemble pompe (1) selon l'une des revendications précédentes, dans lequel la roue à aubes (4) est pourvue sur sa surface d'un motif caractéristique (11) qui peut être détecté par le capteur (7) dans une position de rotation déterminée de la roue à aubes (4), le motif (11) étant dans ce cas de préférence un motif 2D ou un motif 3D.

7. Ensemble pompe (1) selon l'une des revendications précédentes, dans lequel un moyen d'éclairage est en outre prévu dans et/ou sur le carter de pompe (3) ou sur le capteur (7) lui-même, afin d'éclairer une zone de détection du capteur (7).

8. Ensemble pompe (1) selon la revendication 7, dans lequel le moyen d'éclairage est configuré pour émettre des impulsions lumineuses de manière intermittente, les impulsions lumineuses émises de manière intermittente étant synchronisées avec une vitesse de rotation de la pompe.

9. Ensemble pompe (1) selon l'une des revendications précédentes, comprenant en outre une unité de commande (9) reliée au capteur (7) et configurée pour évaluer des données obtenues par le capteur (7) et, sur la base de celles-ci, déduire une situation de défaut, en particulier un blocage, la formation d'une pointe, une cavitation, une détérioration mécanique et/ou des vibrations.

10. Procédé d'identification d'une situation de défaut dans un ensemble pompe (1) selon l'une des revendications précédentes, dans lequel, dans le procédé :
le capteur (7), en particulier un capteur 2D ou 3D, génère des acquisitions de la roue à aubes (4), et les données de la roue à aubes (4) générées par le capteur (7) sont évaluées par une unité de commande (9) afin de déduire la situation de défaut, en particulier un blocage, la formation d'une pointe, une cavitation, une détérioration mécanique et/ou des vibrations,
**caractérisé**
**en ce que** la surface du capteur (7) est rincée pour éliminer activement des résidus s'accumulant devant la surface du capteur, et/ou
**en ce qu'**un boîtier extérieur ou une surface de capteur du capteur 3D (7) est soumis à un mouvement de secousse par ultrasons de manière à ce qu'une couche de salissure déposée sur celui-ci soit désagrégée ou que la formation d'une couche de salissure soit impossible, et/ou
**en ce que** de l'eau pure est ajoutée du côté aspiration de l'ensemble pompe (1) peu avant la création des acquisitions de la roue à aubes (4), afin de réduire une éventuelle turbidité du liquide présent dans l'environnement de la roue à aubes (4) au moment des acquisitions.

11. Procédé selon la revendication 10, dans lequel les acquisitions du capteur (7) reproduisent une zone déterminée de la roue à aubes (4) qui est pourvue d'un motif caractéristique (11), de sorte que l'évaluation de la présence d'un cas de blocage ou de la formation d'une pointe peut être effectuée plus facilement, étant donné que dans ce cas, le motif (11) n'est pas du tout reconnaissable ou n'est reconnaissable qu'avec difficulté.

12. Procédé selon l'une des revendications 10 ou 11, **caractérisé en ce que** la création des acquisitions est synchronisée avec la vitesse de rotation de la roue à aubes (4) pour détecter en continu, de préférence de manière intermittente, une section déterminée ou plusieurs sections déterminées de la roue à aubes (4) à l'aide du capteur (7).

13. Procédé selon l'une des revendications 10 à 12 précédentes, dans lequel les activités d'un personnel d'exploitation de l'ensemble pompe (1), comme la mise en marche ou l'arrêt de l'ensemble pompe (1) et/ou un accusé de réception de défaut, sont surveillées à l'aide du capteur (7) pour obtenir un état réel plus fiable.
